# EUROPEAN PATENT SPECIFICATION

(11) **EP 2 999 512 B1**
(45) Date of publication and mention of the grant of the patent: **05.12.2018**
(21) Application number: 14733335.5
(22) Date of filing: 22.05.2014
(51) Int. Cl.: A61M 39/10

(54) **LOCKING CANNULA FOR MEDICAL LINES**
VERRIEGELUNGSKANÜLE FÜR MEDIZINISCHE LEITUNGENVERRIEGELUNGS
CANULE DE VERROUILLAGE POUR CONDUITES MÉDICALES

(30) Priority: 23.05.2013 IT TO20130411
(43) Date of publication of application: 30.03.2016
(73) Proprietor: Industrie Borla S.p.A., 10024 Moncalieri (Torino) (IT)
(72) Inventor: GUALA, Gianni, 10133 Torino (IT)
(74) Representative: Buzzi, Franco
(86) International application number: PCT/IB2014/061627
(87) International publication number: WO 2014/188372

(56) References cited:
- US-A- 5 810 792
- US-A- 6 142 446
- US-A1- 2007 073 270
- US-B2- 7 140 401

## Description

### Field of the invention

The present invention relates to a cannula for medical lines, comprising a tubular body having a cannula appendage extending axially therefrom, intended to be inserted, for example, through a pre-fissured sealing elastic diaphragm of a medical connector.

In particular, the invention relates to any such cannula provided with a locking device for its temporary anchoring to the connector carrying the sealing diaphragm, following its crossing by the cannula appendage.

### State of the prior art

From the U.S.A. document DES-321,252 a cannula of the type defined above is known, in which the locking device is formed by a pair of elongated anchoring wings arranged on opposite sides of the body and including respective manoeuvring parts and respective engagement parts, with one arranged along the prolongation of the other. The engagement parts of the two anchoring wings extend along the opposite sides of the cannula appendage, at a certain distance and generally parallel thereto and have respective gripping teeth ends.

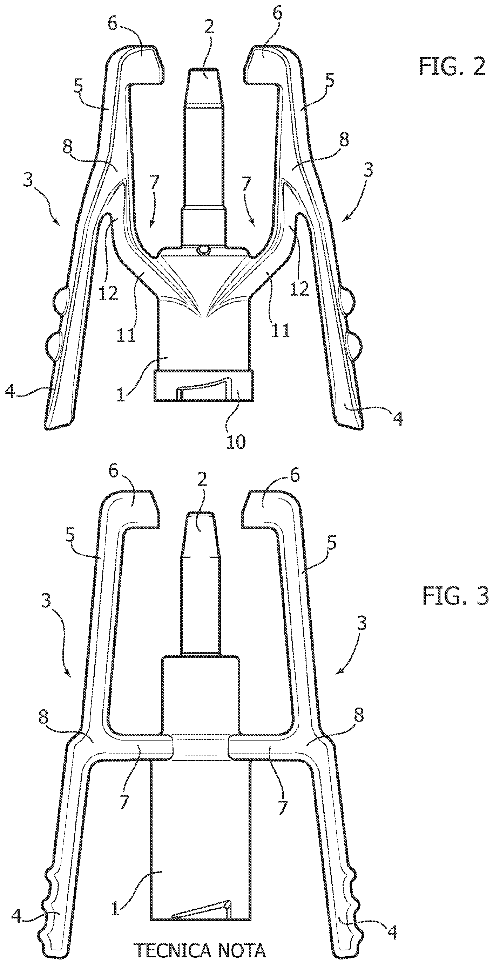

This known arrangement is represented in Figure 3 of the accompanying drawings, in which the body and the cannula appendage are indicated with 1 and 2, respectively, and the anchoring wings with 3. The references 4 and 5 indicate, respectively, the manoeuvring part and the engagement part of each anchoring wing 3, and 6 indicates the gripping teeth of the engagement parts 5.

The body 1 with the cannula appendage 2 and the anchoring wings 3 are integrally formed in a single piece of moulded plastic material, typically polycarbonate or the like, and the connection between the body 1 and each anchoring wing 3 is created by means of a respective elastically deformable arm 7.

The arrangement is such that the mutual approaching of the manoeuvring parts 4 produces, due to the elastic deformation of the arms 7, a rotation of the two wings 3, corresponding to the mutual spacing-apart and therefore the opening of the engagement parts 5 to allow, during use, the application and removal of the cannula relative to the connector equipped with the pre-fissured sealing diaphragm. The release of these manoeuvring parts 4 causes the return of the arms 7 to their undeformed condition, and the subsequent rotation of the wings 3, corresponding to the mutual re-approaching of the engagement parts 5.

As is clearly visible in Figure 3, in this known cannula, the elastically deformable arms 7 are radially orientated with respect to the body 1, and are connected to the relative anchoring wings 3 essentially orthogonal thereto, at the transition areas between the manoeuvring parts 4 and the engagement parts 5, indicated with 8.

In use, the actuation of the manoeuvring parts 4 for achieving the mutual approaching, carried out by the thumb and the index finger of one hand, produces a localized bending deformation almost solely in the respective elastically deformable arms 7, as the two wings 3 essentially rotate rigidly i.e. without substantial deformation in the transition areas 8 between the manoeuvring parts 4 and the engagement parts 5.

This results in a twofold drawback.

Firstly, the force, which must be applied by the user's thumb and index finger on the manoeuvring parts 4, to achieve the necessary spacing-apart between the gripping teeth 6 of the engagement parts 5 is considerable, if not excessive.

Secondly, the localized elastic deformation of the arms 7 often causes premature breaking, which evidently implies a poor reliability of the cannula. An analogous cannula, presenting similar drawbacks, is described in US5810792.

### Summary of the invention

The object of the present invention is to overcome the aforesaid drawbacks and to produce a more robust locking cannula, which is therefore more reliable, as well as being operable with an appreciably reduced manual force.

According to the invention, this object is achieved thanks to a locking cannula of the type defined in the preamble of claim 1, whose unique characteristic lies in the fact that the aforesaid elastically deformable arms each have a first portion joining to the body, obliquely orientated relative to the cannula appendage, and a second portion joining to the engagement part of the respective anchoring wing, essentially orientated parallel thereto.

Thanks to this solution idea, the force applied to the manoeuvring parts of the anchoring wings of the cannula according to the invention, to create the opening of the engagement parts, is appreciably reduced compared to the case of the known cannula described above. The unique and specific configuration of the two arms, which connect the anchoring wings to the body of the cannula, also allows avoiding the concentration of bending forces, which in practice are broken down in part along the first joining portion of each arm to the body, and in part in the connecting area between each arm and the engagement part of the respective anchoring wing, thus producing a double elastic deformation. The reduction and the distribution of the forces applied, which are in fact one order of magnitude lower than those of the known cannula described above, also ensure an appreciably improved reliability of the cannula according to the invention, thanks to the substantial suppression of the risk of not only premature breaking, but also following repeated manoeuvring cycles of the locking device.

According to a preferred embodiment of the invention, the second joining portion of each arm to the engagement part of the respective anchoring wing, essentially extends along the prolongation of that engagement part, the length of which is considerably less than the length of the manoeuvring part.

### Brief description of the drawings

The invention will now be described in detail with reference to the accompanying drawings, provided purely by way of non-limiting example, in which:
Figure 1 is a perspective view of a locking cannula according to the invention,
Figure 2 is an elevational side view of the cannula, and
Figure 3 is a view analogous to Figure 2 that represents the locking cannula according to the prior art, described above.

### Detailed description of the invention

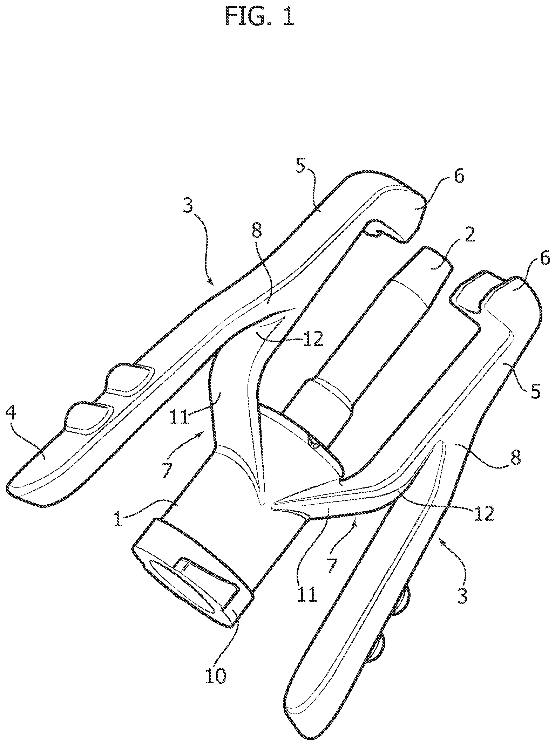

Referring to Figures 1 and 2, the locking cannula according to the invention consists of a single piece of moulded plastic material, typically polycarbonate or the like, whose general configuration is similar to that previously described with reference to the cannula according to the prior art, illustrated in Figure 3. Below, the same numerical references for the identical or similar parts to those already described will therefore be used.

The cannula comprises a tubular body 1, which forms a female luer-lock connector 10 at one end for coupling to a male luer-lock connector of a medical line, and the other end has an axial cannula appendage 2, intended to be introduced through a pre-fissured elastic diaphragm of a medical connector, for example, of the Y-shaped type.

For achieving the locking of the cannula appendage 2, following its insertion into the medical connector, a pair of anchoring wings 3 is provided, arranged on opposite sides of the body 1, and connected thereto by means of respective elastically deformable arms 7.

Each anchoring wing 3 has an elongated shape with a respective manoeuvring part 4, extending at the side of the body 1, and a respective engagement part 5, extending at the side of the cannula appendage 2, generally parallel and spaced with respect thereto. The free end of each engagement part 5 has a gripping tooth 6 for engaging with the wall of the medical connector that, in use, is coupled with the cannula appendage 2.

According to the unique characteristic of the invention, the elastically resilient arms 7 have a configuration adapted to allow both a surprisingly reduced force required for the manual operation of the anchoring wings 3, where the relative engagement parts 5 must be spaced apart from each other by means of the mutual approaching of the relative manoeuvring parts 4, and a drastic reduction in the risk of breaking, so as to improve the reliability of the cannula.

According to the invention, each elastically deformable arm 7 has a first portion 11 joined to the body 1, which is obliquely orientated with respect to the body 1 itself to the cannula appendage part 2, and a second portion 12 that joins the engagement part 5 of the respective anchoring wing 3. The second portion 12 is arranged on the prolongation of this engagement part 5 and is therefore essentially orientated parallel thereto. It is therefore angled with respect to the first portion 11.

The transition areas 8 between the manoeuvring parts 4 and the engagement parts 5 of the anchoring wings 3 are therefore moved, with respect to the cannula according to the prior art of Figure 3, from the cannula appendage part 2 and, for example, essentially arranged at an axially median area of this.

Again comparing with the known cannula of Figure 3, the engagement parts 5 of the anchoring wings 3 have a shorter length, while the manoeuvring parts 4 have a substantially greater length, for example almost double, than that of the engagement parts 5.

Experimental tests conducted by the Applicant have allowed the verification, in comparison with the cannula according to the prior art, that the force to be applied by the thumb and index finger of the user's hand to operate the mutual approaching of the manoeuvring parts 4, and therefore the opening of the engagement parts 5, is drastically reduced, and that the forces thus applied, and the resulting elastic deformations are distributed into essentially homogeneous measures between the arms 7 and the manoeuvring parts 4 in the vicinity of the transition areas 8. In this way, a concentration of forces on the arms 7 is prevented, and in particular at the relative attachment ends to the body 1, drastically reducing, if not eliminating, the risk of breaking, even after repeated actuation cycles.

Naturally, the details of construction and the embodiments may be varied with respect to what is described and illustrated, without departing from the scope of the present invention as defined in the following claims.

## Claims

1. A locking cannula for medical lines comprising a tubular body (1) having a cannula appendage (2) extending axially therefrom, and a pair of elongated anchoring wings (3) arranged on opposite sides of the body (1) and including respective manoeuvring parts (4) and respective engagement parts (5), said anchoring wings (3) being connected to the body (1), between said manoeuvring parts (4) and said engagement parts (5), through respective elastically deformable arms (7) so that the mutual approaching of said manoeuvring parts (4) produces mutual spacing-apart of said engagement parts (5), **characterized in that** said elastically deformable arms (7) each have a first portion (11) joined to the body (1), which is obliquely orientated to the side of the cannula appendage (2), and a second portion (12) joined to the engagement parts (5) of the respective anchoring wing (3), which is essentially orientated parallel thereto.

2. A cannula according to claim 1, **characterized in that** the second portion (12) of each arm (7) extends along the prolongation of the engagement part (5) of the respective anchoring wing (3).

3. A cannula according to claim 1 or claim 2, **characterized in that** the manoeuvring part (4) of each arm (7) has a substantially greater length than the respective engagement parts (5).

## Patentansprüche

1. Verriegelungskanüle für medizinische Leitungen, die einen röhrenförmigen Körper (1) mit einem sich axial davon erstreckenden Kanülenfortsatz (2) und ein Paar lang gestreckter Verankerungsflügel (3), die auf gegenüberliegenden Seiten des Körpers (1) angeordnet sind und jeweilige Handhabungsteile (4) und jeweilige Eingriffsteile (5) aufweisen, umfasst, wobei die Verankerungsflügel (3) zwischen den Handhabungsteilen (4) und den Eingriffsteilen (5) über jeweilige elastisch verformbare Arme (7) mit dem Körper (1) verbunden sind, so dass das Annähern der Handhabungsteile (4) aneinander ein Beabstanden der Eingriffsteile (5) voneinander bewirkt, **dadurch gekennzeichnet, dass** die elastisch verformbaren Arme (7) jeweils einen mit dem Körper (1) verbundenen ersten Abschnitt (11), der schräg zur Seite des Kanülenfortsatzes (2) ausgerichtet ist, und einen mit den Eingriffsteilen (5) des jeweiligen Verankerungsflügels (3) verbundenen zweiten Abschnitt (12), der im Wesentlichen parallel dazu ausgerichtet ist, aufweisen.

2. Kanüle nach Anspruch 1, **dadurch gekennzeichnet, dass** der zweite Abschnitt (12) jedes Arms (7) sich entlang der Verlängerung des Eingriffsteils (5) des jeweiligen Verankerungsflügels (3) erstreckt.

3. Kanüle nach Anspruch 1 oder Anspruch 2, **dadurch gekennzeichnet, dass** das Handhabungsteil (4) jedes Arms (7) eine im Wesentlichen größere Länge aufweist als die jeweiligen Eingriffsteile (5).

## Revendications

1. Canule de verrouillage pour conduites médicales comprenant un corps tubulaire (1) ayant un appendice de canule (2) s'étendant de manière axiale à partir de ce dernier, et une paire d'ailes d'ancrage allongées (3) agencées sur les côtés opposés (1) et comprenant des parties de manoeuvre (4) respectives et des parties de mise en prise (5) respectives, lesdites ailes d'ancrage (3) étant raccordées au corps (1), entre lesdites parties de manoeuvre (4) et lesdites parties de mise en prise (5), par le biais de bras élastiquement déformables (7) respectifs de sorte que le rapprochement mutuel desdites parties de manoeuvre (4) produit l'espacement mutuel desdites parties de mise en prise (5), **caractérisée en ce que** lesdits bras élastiquement déformables (7) ont chacun une première partie (11) assemblée au corps (1), qui est orientée de manière oblique vers le côté de l'appendice de canule (2), et une seconde partie (12) assemblée aux parties de mise en prise (5) de l'aile d'ancrage (3) respectives, qui est essentiellement orientée parallèlement à cette dernière.

2. Canule selon la revendication 1, **caractérisée en ce que** la seconde partie (12) de chaque bras (7) s'étend le long du prolongement de la partie de mise en prise (5) de l'aile d'ancrage (3) respective.

3. Canule selon la revendication 1 ou la revendication 2, **caractérisée en ce que** la partie de manoeuvre (4) de chaque bras (7) a une longueur sensiblement supérieure aux parties de mise en prise (5) respectives.
